# EUROPÄISCHE PATENTSCHRIFT

(11) **EP 3 027 012 B1**
(45) Veröffentlichungstag und Bekanntmachung des Hinweises auf die Patenterteilung: **06.12.2017**
(21) Anmeldenummer: 14744462.4
(22) Anmeldetag: 24.07.2014
(51) Int. Cl.: A01K 11/00, A61B 10/02

(54) **VERFAHREN UND VORRICHTUNG ZUR ANBRINGUNG EINER OHRMARKE**
METHOD AND DEVICE FOR ATTACHING AN EAR TAG
PROCÉDÉ ET DISPOSITIF D'APPLICATION D'UNE MARQUE AURICULAIRE

(30) Priorität: 30.07.2013 DE 102013012554
(43) Veröffentlichungstag der Anmeldung: 08.06.2016
(73) Patentinhaber: Prionics AG, 8952 Schlieren (CH)
(72) Erfinder: BERNER, Alexander, 81479 München (DE)
(74) Vertreter: Weber, Birgit
(86) Internationale Anmeldenummer: PCT/EP2014/002022
(87) Internationale Veröffentlichungsnummer: WO 2015/014461

(56) Entgegenhaltungen:
- WO-A1-2007/013820
- WO-A2-2006/000869
- US-A- 4 597 208
- US-A1- 2010 210 011
- US-A1- 2011 270 267

## Beschreibung

Die Erfindung betrifft ein Verfahren nach dem Oberbegriff des Anspruches 1 und eine Vorrichtung nach dem Oberbegriff des Anspruches 7.

Ohrmarken zur Kennzeichnung von Nutztieren haben sich weltweit durchgesetzt. Man findet nur noch sehr wenige Kühe, Ziegen oder Schafe, die nicht mit Ohrmarken ausgerüstet sind. Diese Ohrmarken dienen primär der Kennzeichnung zu Zwecken der Organisation und Erfassung des Viehbestandes, der Zuordnung zu den Eigentümern sowie der Rückverfolgbarkeit der Nutztiere.

Dazu gehört unabdingbar die Sicherung gegen Betrugsversuche. Es muss also insbesondere verhindert werden, dass eine Ohrmarke von einem Tier abgenommen und auf ein anderes übertragen wird.

In der US 4 597 208 A ist eine zweiteilige Ohrmarke dargestellt. Eine Lochplatte mit einem Loch und eine Dornplatte mit einem Dorn werden verbunden, indem der Dorn durch das Ohr gestanzt und in das Loch gesteckt wird um dort mit Hinterschnitt verriegelt zu werden. Zum Manipulationsschutz ist hierbei das Loch auf der Außenseite mit einer stabilen Kappe abgedeckt, die Manipulationsversuche verhindert oder durch offensichtliche Beschädigungsmerkmale zur Anzeige bringt.

Insbesondere zur Bekämpfung von Tierseuchen sowie für die Genanalyse zu züchterischen Zwecken werden flächendeckende Laboruntersuchungen von Nutztieren durchgeführt. Zur Arbeitsvereinfachung empfiehlt es sich dabei, die Entnahme einer Gewebeprobe am Tier mit der Kennzeichnung zu verbinden. Eine dafür geeignete Ohrmarke ist in der gattungsgemäßen US 2010/0210011 A1 dargestellt. Diese Ohrmarke entspricht grundsätzlich der Konstruktion der zuvor beschriebenen bekannten Konstruktion, weist jedoch zusätzlich einen Entnahmekopf auf, der in Einstechrichtung des Dornes vor diesem befestigt ist und mit einer vorderen Ringschneide beim Durchstechen des Ohres aus diesem eine Probe entnimmt. Nachteilig dabei ist allerdings, dass der Entnahmekopf nach Einrasten des Dornes in dem Loch, durch das Loch hindurchgesteckt und hinter diesem im Freien angeordnet ist. Ein manipulationsverhinderndes Abdecken der Lochrückseite wie dies die eingangs genannte bekannte Schrift zeigt, ist hier nicht möglich, da die Rückseite des Loches frei zugänglich bleiben muss, um die Probe von dort Entnehmen zu können.

Aus diesen Gründen weist der bekannte gattungsgemäße Stand der Technik erhebliche Sicherheitsmängel auf. Es ist zum Beispiel relativ leicht möglich, von der Rückseite des Loches her auf den Dorn zu drücken und diesen wieder durch das Loch zurück zu drücken und dann erneut zu verwenden.

Die Aufgabe der vorliegenden Erfindung besteht daher darin, bei einer gattungsgemäßen Vorrichtung die Manipulationssicherheit zu erhöhen.

Diese Aufgabe wird mit den Merkmalen der Kennzeichnungsteile der Ansprüche 1 und 7 gelöst.

Erfindungsgemäß werden bei der gattungsgemäßen Vorrichtung der Entnahmekopf und der Dorn derart zusammenarbeitend gestaltet, dass sich durch ihr Einwirken auf die Probe eine Bewegungsbahn für diese ergibt, die wenigstens in einem Bereich eine quergerichtete Bewegungskomponente aufweist Damit ergibt sich eine Bewegung der Probe die diese aus der Bewegungsbahn des Dornes zur Seite entfernt. Somit wird vermieden, dass der Dorn die Probe mit durch das Loch nimmt. Es kann bei einer im Wesentlichen herkömmlichen Vorrichtung der Dorn in dem Loch verankert werden, das in beliebiger Weise manipulationsverhindernd ausgebildet, z.B. rückseitig mit einer Schutzkappe versehen sein kann. Hierdurch wird die Entnahme der Probe aber nicht behindert, da diese aufgrund ihrer quergerichteten Bewegungskomponente nicht in die Schutzkappe gelangt, sonder zu einem anderen Ort befördert wird, an dem sie leicht zugänglich bleibt.

Es können hier also alle herkömmlichen Techniken zur Manipulationsverhinderung, wie zum Beispiel Abdeckkappen für das Loch verwendet werden. Erfindungsgemäß können lange eingeführte und behördlich zugelassene Ohrmarkenkonstruktionen verwendet werden, die eine Probeentnahme bei Aufrechterhaltung eines hohen Manipulationsschutzes ermöglichen.

Vorteilhafte Verfahrensvarianten ergeben sich aus den Ansprüchen 2 bis 6.

Eine vorteilhafte Vorrichtung zur Durchführung des erfindungsgemässen Verfahrens ergibt sich aus Anspruch 7.

Vorteilhaft sind die Merkmale des Anspruches 8 vorgesehen. Hierdurch lässt sich das Abtrennen besonders einfach z.B. nach Art einer Trennschere in die erfindungsgemässe Vorrichtung integrieren, wobei vorzugsweise gemäss Anspruch 9 hiermit z.B. durch Ausnutzung der Bewegung der Trennglieder auch eine unkomplizierte Erzeugung der Querkomponente möglich ist.

Vorteilhaft gemäß den Ansprüchen 10 bis 12 können die Trennglieder durch den Dorn und einen Behälter gebildet werden, der z.B. der Container zur Verschickung der Probe sein kann und der auch durch den Entnahmekopf gebildet sein kann. Dadurch lässt sich die Konstruktion unter Vermeidung zusätzlicher Teile stark vereinfachen. Es besteht sogar die Möglichkeit, das Loch des Lochteiles als stehendes Trennglied zu nutzen.

Vorteilhaft gemäß Anspruch 13 weist der Dorn eine Schrägfläche auf, die gut beim Trennvorgang einsetzbar ist und die sich auch zur Erzeugung der Querbewegung eignet.

Vorteilhaft ist dabei gemäss Anspruch 14 die Fläche als Kegelfläche ausgebildet. Damit liegt eine rundum symmetrische Schrägfläche vor. Vorteilhaft hierbei ist auch die Verwendbarkeit herkömmlicher Ohrmarken mit kegelförmigem Dorn.

Bei der abtrennenden Kollision zwischen Dorn und Entnahmekopf kann gemäss Anspruch 15 eine Schneide das Abtrennen durch einen Schneideffekt erleichtern.

Alternativ oder zusätzlich kann gemäß Anspruch 16 eine Nadelanordnung das Abtrennen erleichtern. Diese kann das Ohr im Bereich der Probe am Entnahmekopf fixieren, so dass bei der Relativbewegung gegenüber dem Dorn ein Abtrennen durch Abreißen erfolgt. Hiermit lässt sich auch das Abdrängen des Ohres ohne Gewinnung einer Probe verhindern. Die Nadelanordnung muss nicht unbedingt wirkliche Nadeln aufweisen. Sie kann auch nur aus einem rauen Oberflächenbereich bestehen, der bei ausreichender Andruckkraft die Probe sicher hält.

Vorteilhaft gemäß Anspruch 17 weist der Entnahmekopf einen Schlitz auf, der auch als Sollbruchstelle ausgebildet sein kann und der so gelegt ist, dass er nach Hindurchlaufen des Dornes bei dessen anschließender seitlicher Entnahmebewegung den Dornschaft leicht und ohne Zerstörungen passieren lässt.

Eine alternative Konstruktion ergibt sich gemäß Anspruch 18 wonach der Entnahmekopft in konventioneller Weise vor dem Dorn angeordnet ist und somit von diesem auf das Loch zubewegt wird. Die ablenkende Einrichtung im Bereich des Lochteiles sorgt jedoch dafür, dass der Entnahmekopf mit der darin liegenden Probe vor Erreichen des Lochteiles seitlich abgelenkt wird.

Eine weitere alternative Ausführungssform ergibt sich gemäß Anspruch 19. Hier läuft der Entnahmekopf auf einer zweiten Bewegungsbahn die die erste Bewegungsbahn im Punkt der Durchdringung des Ohres kreuzt.

Ein erfindungsgemässer Entnahmekopf ergibt sich aus Anspruch 20.

In den Zeichnungen ist die Erfindung beispielsweise und schematisch dargestellt. Es zeigen:
- Figuren 1.1 bis 1.7: verschiedene Phasen der Relativbewegung zwischen Dorn und Entnahmekopf bei einer ersten Ausführungsform der Erfindung,
- Figuren 2.1 bis 2.6: verschiedene Phasen der Relativbewegung zwischen Dorn und Entnahmekopf bei einer zweiten Ausführungsform der Erfindung,
- Figuren 3.1 bis 3.4: verschiedene Phasen der Relativbewegung zwischen Dorn und Entnahmekopf bei einer dritten Ausführungsform der Erfindung und
- Figuren 4.1 bis 4.3: verschiedene Phasen der Relativbewegung zwischen Dorn und Entnahmekopf bei einer vierten Ausführungsform der Erfindung.

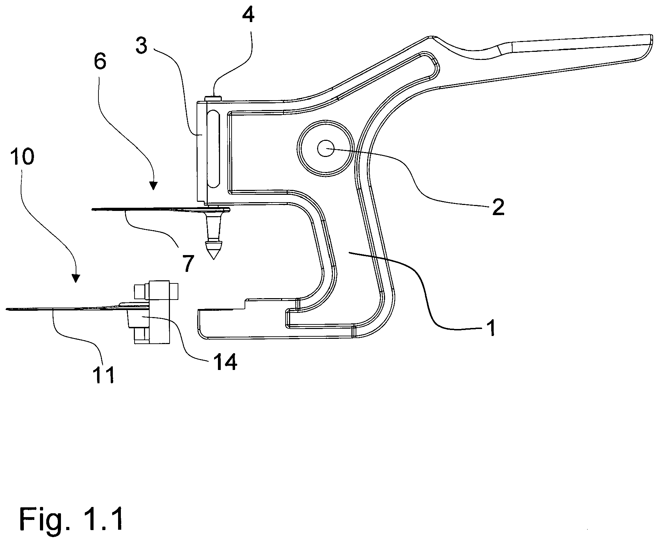

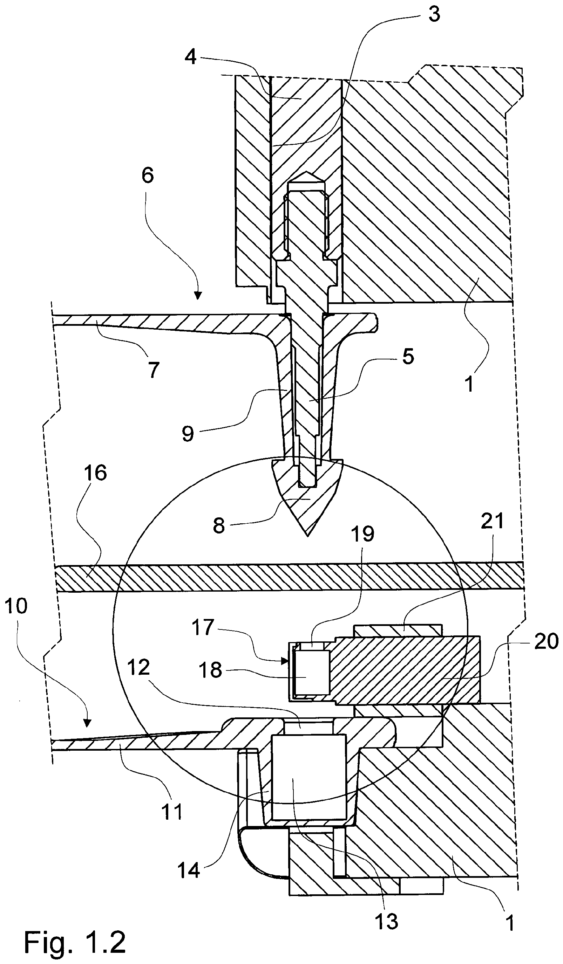

Die Figuren 1.1 bis 1.7 zeigen eine erste Ausführungsform der Erfindung, wobei in Figur 1.1 eine seitliche Ansicht der kompletten Vorrichtung mit einer Zange 1 dargestellt ist. Diese Zange besteht aus zwei um eine Achsbohrung 2 schwenkbar aneinander gelagerten Teilen, von den eines zur Vereinfachung der Zeichnung weggelassen ist. Im dargestellten Teil der Zange 1 ist in einer Führungsbohrung 3 ein Bolzen 4 verschiebbar gelagert, der, wie Figur 1.2 zeigt, an seinem unteren Ende einen Dornstift 5 trägt. Der Bolzen 4 ist in nicht dargestellter Weise über eine entsprechende Kinematik mit dem nicht dargestellten zweiten Teil der Zange 1 verbunden und wird durch zangenartiges Verschwenken der beiden Zangenteile angetrieben.

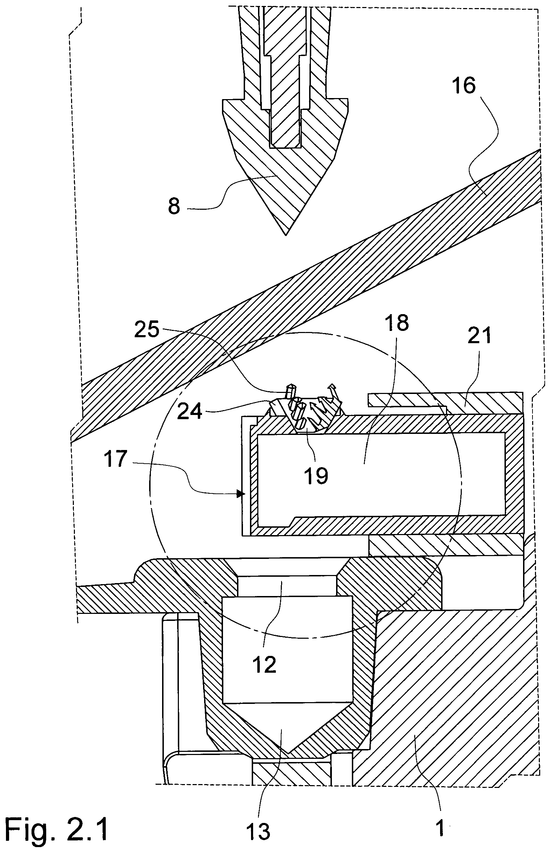

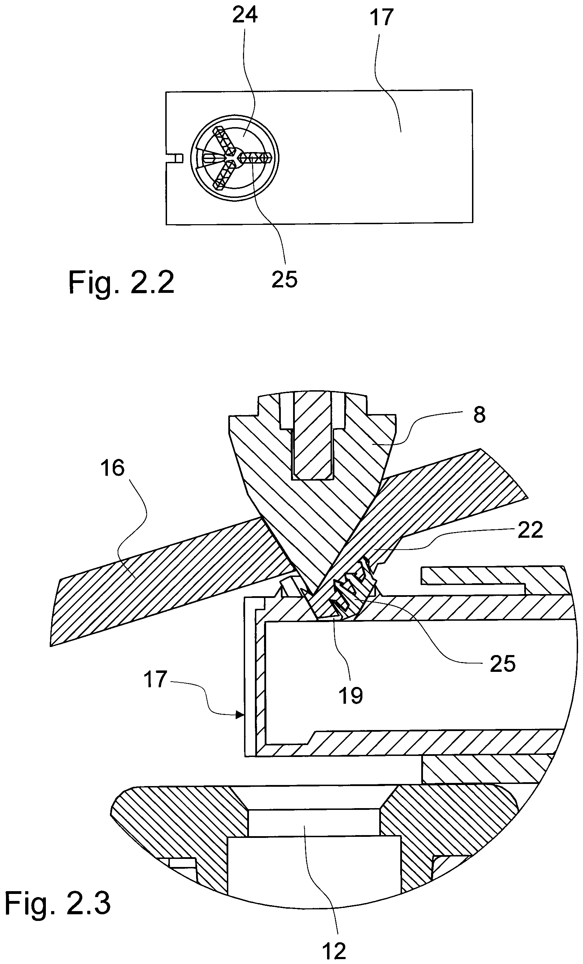

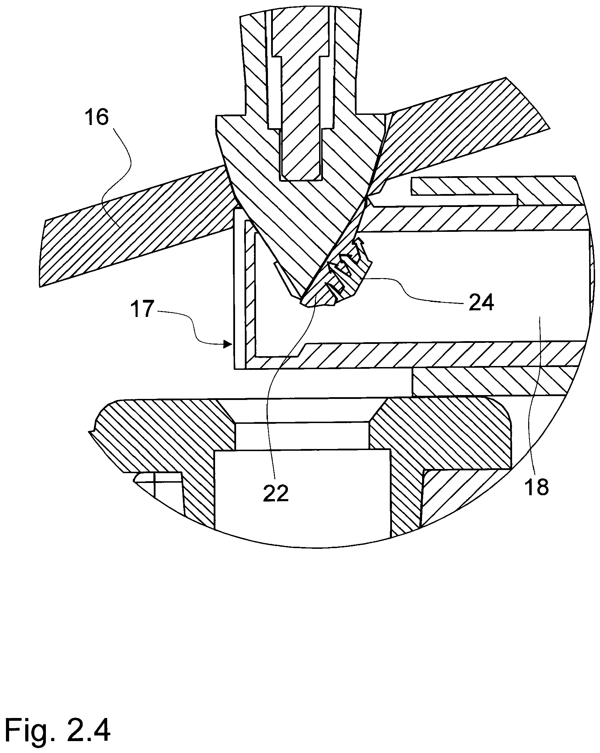

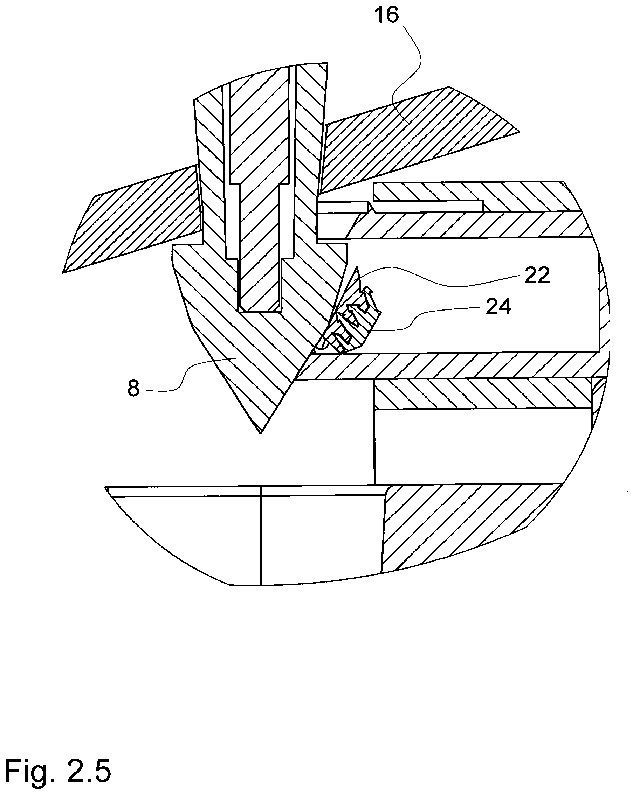

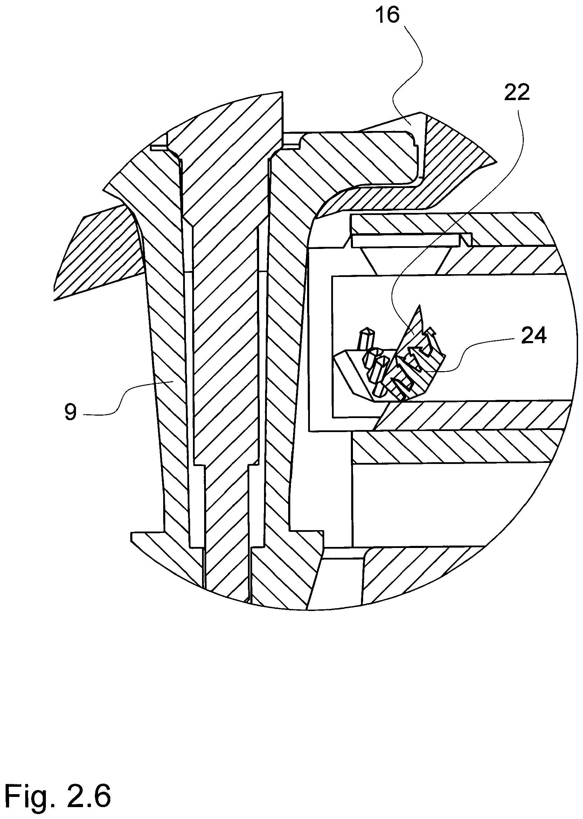

Auf dem in Figur 2 dargestellten Dornstift 5 ist ein Dornteil 6 einer Ohrmarke aufgesteckt, wie dies die Figur 1.2 zeigt. Der Dornteil 6 weist eine Dornplatte 7 auf, mit der ein kegelförmig geformter Dorn 8 über einen Dornschaft 9 verbunden ist. Der Dornschaft ist zur Aufnahme des Dornstiftes 1 hohl, wie dies die Figur 1.2 zeigt.

Ein Lochteil 10 bildet den zweiten Teil der Ohrmarke. Er weist eine Lochplatte 11 auf, in der, wie Figur 1.2 zeigt, ein Loch 12 angeordnet ist. Auf der dem Dorn 8 gegenüberliegenden Seite des Loches 12 ist dieses von einem eine Kammer 13 umschließenden Gehäuse 14 manipulationssicher verschlossen. Die Kammer 13 weitet sich gegenüber dem Durchmesser des Loches 12, so dass der Dorn 8 nach Durchdringen des Loches 12 mit seinem Rand 15 hinter den Rand des Loches 12 einrasten kann. Damit werden die Teile 6,10 der Ohrmarke aneinander gesichert. Ein manipulierendes Zugreifen zum Dorn 8 wird durch die Wand 14 der Kammer 13 verhindert.

In Figur 1.2 ist auch ein Ohr 16, zum Beispiel das Ohr einer Kuh, dargestellt, an dem die Ohrmarke 6, 7 befestigt werden soll. Dabei soll auch eine Gewebeprobe entnommen werden. Das ist am Gegenstand der Figur 1.2 in aufeinander folgenden Bewegungsphasen dargestellt, die in den Figuren 1.2 bis 1.7 gezeigt sind.

Unterhalb des Ohres 16, in der Nähe des Loches 12, ist ein Entnahmekopf 17 angeordnet, wie dies in Figur 1.2 zu sehen ist. Der Entnahmekopf 17 bildet einen rundum geschlossenen Entnahmeraum 18 aus, der auf der oberen, zum Dorn 8 hin liegenden Seite eine Entnahmeöffnung 19 aufweist. Der Entnahmekopf 17 ist mit einem Kolben 20 in einer rohrförmigen Führung 21 an der Zange 1 in Richtung quer zur Achse des Dornes 8 verschiebbar gelagert.

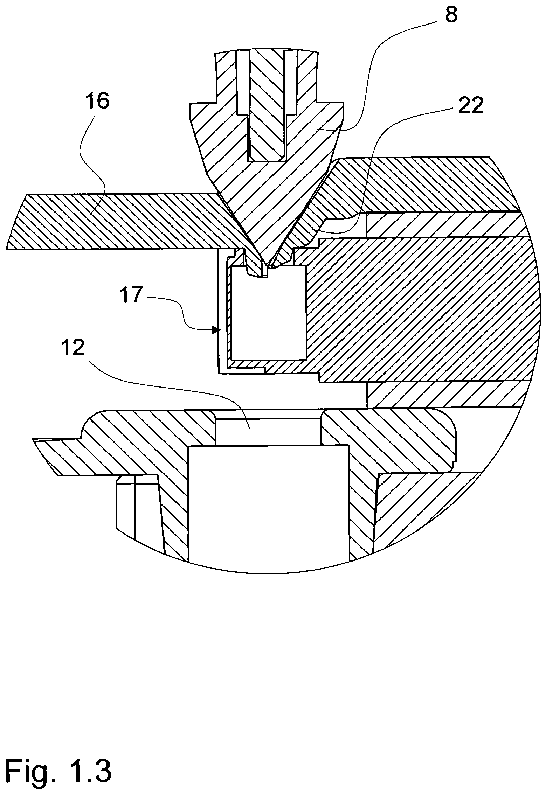

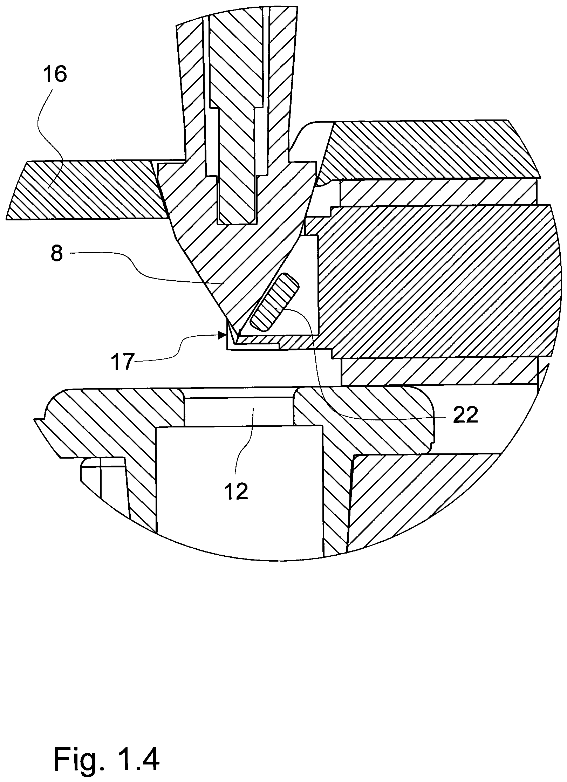

Zu Beginn des Vorganges zur Behandlung eines Ohres werden zunächst die Ohrmarkenteile 6 und 10 an der Zange 1, so wie das die Figur 1.2 zeigt, angebracht. Dabei wird auch der Entnahmekopft 17 in der in Figur 1.2 gezeigten Weise montiert. Der Dorn 8 steht weit oben in der Stellung gemäß Figur 1.2. Das Ohr ist unter dem Dorn 8 angeordnet. Nun wird durch Betätigung der Zange der Dorn 8 in Richtung nach unten angetrieben. Er drückt, wie Figur 1.3 zeigt, zunächst das Ohr 16 nach unten bis gegen den Entnahmekopf 17 und schneidet dabei in das Ohr 16 ein, wie Figur 1.3 zeigt. Dabei wird aufgrund der Formgebung der Öffnung 19 des Entnahmekopfes 17 und der des Dornes 8, so wie in Figur 1.3 angedeutet, eine Probe 22 vom Gewebe des Ohres 16 abgetrennt und, wie das die Figur 1.4 zeigt, in dem Entnahmeraum 18 abgelegt.

Es schneidet hier also die konische Oberfläche des Dornes 8 als ein Trennglied gegen die obere Randkante der Öffnung 19, als zweites Trennglied, wodurch die Probe 22 vom Ohr 16 abgetrennt wird. Diese Abtrennung kann unvollständig sein und einen Verbindungsstrang zwischen der Probe 22 und dem Ohr 16 belassen. Dieser kann dann in einer endgültigen Trennung zerrissen oder zerschnitten werden. Dazu z.B. kann an der Öffnung 19 eine zweite, stufenförmig unterhalb der oberen Randkante angeordnete Kante vorgesehen sein.

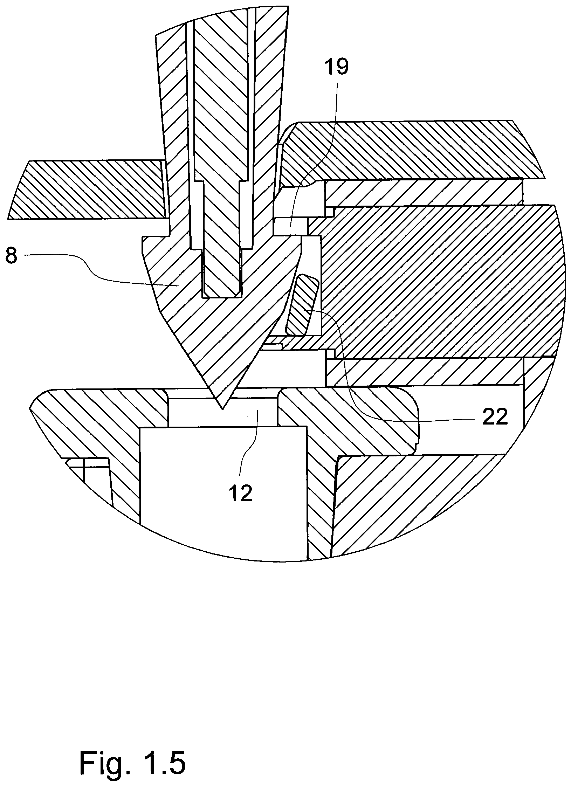

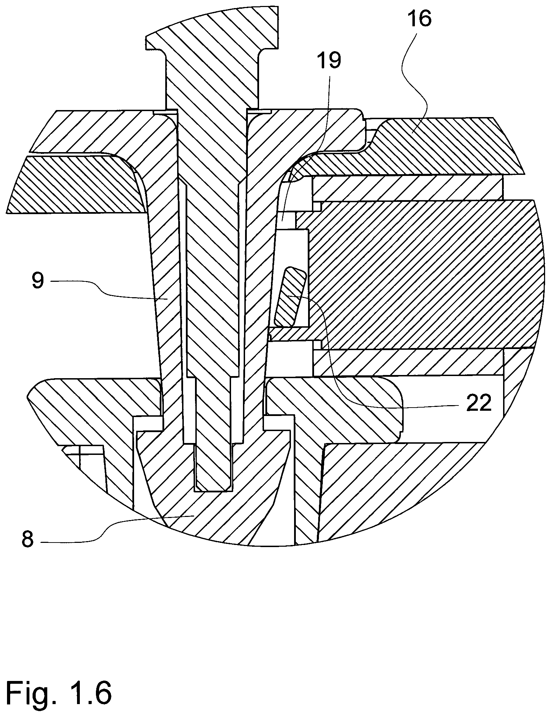

Figur 1.5 zeigt, dass der Dorn 8 bei weiterer Absenkung den Entnahmekopf 17 von oben nach unten durchläuft und in das Loch 12 eindringt. Im nächsten Bewegungsschritt gemäß Figur 1.6 hat der Dorn 8 den untersten Punkt seiner Bewegung erreicht. Er sitzt nun in dem Kammer 13 hinter dem Rand des Loches 12 eingerastet. Die Probe 22 liegt unbehelligt neben dem Dornschaft 9 in dem Entnahmeraum 18.

In de Position der Figur 1.6 ist der Anbringungsvorgang der Ohrmarke durch die sichere Verriegelung dieser beiden Ohrmarkenteile gemäß Figur 1.6 abgeschlossen und ebenso der Entnahmevorgang der Probe 22 die nun an ihrem Zielort im Entnahmeraum 18 liegt.

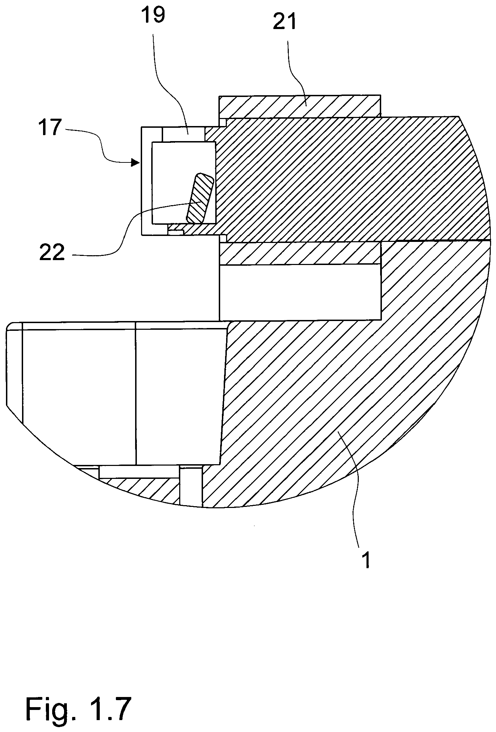

Es werden nun die Ohrmarkenteile 6 und 10 von der Zange entfernt und zwar in Richtung nach links gemäß Figur 1.6. Dabei bleibt jedoch der Entnahmekopf 17 in seiner Führung 21 an der Zange 1. Der Dornschaft 9 wird seitlich aus dem Entnahmeraum 18 herausgezogen. Dazu hat er in seiner in der Figur linken Seitenwand einen Schlitz 23, der sich beim seitlichen Herausziehen des Dornschaftes 9 öffnet, den Dornschaft durchlässt und dann wieder schließt, so wie dass in Figur 1.7 dargestellt ist. Das Material des Entnahmekopfes 17 muss die dafür geeignete Elastizität aufweisen.

Der Entnahmkopf 17 kann nun aus der in Figuren 1.7 dargestellten Position von der Zange 1 abgenommen und zum Beispiel in ein Labor geschickt werden, um dort die gewünschten Analysen vornehmen zu lassen. Dazu wird die Probe 22 vorzugsweise noch in einen Transportcontainer gebracht und luftdicht verschlossen.

Vergleicht man noch einmal die Bewegungsabfolge wie sie in den Figuren 1.2 bis 1.7 dargestellt ist, so ist zu erkennen, dass der Dorn 8 auf einer linearen, in den Figuren von oben nach unten verlaufenden ersten Bewegungsbahn aus der Stellung gemäß Figur 1.2 bis in die Stellung gemäß Figur 1.6 gebracht wird. Die aus dem Ohr 16 abgetrennte Probe 22 liegt zunächst, wenn sie noch im Ohr verbunden ist, genau im Weg des Dornes 8 und wird auch zunächst, wie Figur 1.3 zeigt, in der Bewegungsbahn des Dornes 8 bis in die Entnahmeöffnung 19 mitgenommen. Bei weiterer Bewegung des Dornes 8 wird jedoch die Probe 22, so wie das Figur 1.4 zeigt, nicht mehr weiter in der Bewegungsbahn des Dornes 8 mitgenommen, sondern bleibt, wie dies insbesondere Figur 1.5 zeigt, im Entnahmeraum 18 gehalten. Der Dorn 8 läuft weiter nach unten und bewegt dabei die Probe 22 zur Seite und zwar gemäß den Figuren in Richtung nach rechts. Wie Figur 1.5 im Vergleich mit Figur 1.4 zeigt, wird dabei auch der gesamte Entnahmekopf 17 etwas zur Seite gedrückt. Es ergibt sich also anschließend an den Vorgang des Abtrennens der Probe 22 auf einem Stück der Bewegungsbahn eine seitwärts Bewegung der Probe 22.

Damit wird insbesondere verhindert, dass die Probe vom Dorn 8 in die Kammer 13 unterhalb des Loches 12 mitgenommen wird, wo sie schwer zugänglich wäre.

Die Figuren 2.1 bis 2.6 zeigen eine zweite Ausführungsform der Erfindung, bei der die Bauteile bis auf wenige Ausnahmen mit der der zuvor beschriebenen Ausführungsform übereinstimmen. Es werden soweit möglich dieselben Bezugszeichen verwendet.

Figur 2.1 zeigt eine Stellung entsprechend Figur 1.2. Der Dorn 8 sitzt mit dem Dornschaft 9 auf dem Dornstift 5 und soll nun abwärts durch das Ohr 16 und den Entnahmekopf 17 sowie durch das Loch 12 bis in die Kammer 13 bewegt werden.

Bei der ersten Ausführungsform, wie zum Beispiel in den Figuren 1.3 und 1.4 zu ersehen, hat die Entnahmeöffnung 19 des Entnahmeraumes 18 eine scharfkantigen Rand, der zusammen mit der konischen Schrägfläche des Dornes 8, einen Schneidffekt auf die vom Ohr 16 abzutrennende Probe 22 ausübt. Die Probe 22 wird also bei der ersten dargestellten Ausführungsform zumindest überwiegend schneidend abgetrennt.

Bei der zweiten Ausführungsform der Figuren 2.1 bis 2.6 soll weniger abgeschnitten, vielmehr abgerissen werden. Dazu ist die Öffnung 19 des Entnahmeraumes 18 wie dies Figur 2.1 zeigt, mit einem trichterförmigen Kragen 24 versehen, den Figur 2.2 in Draufsicht zeigt. An drei Stellen seines Umfanges, (siehe Figur 2.2) ist der Kragen 24 jeweils mit einer radial angeordneten Reihe von Nadeln 25 versehen. Am deutlichsten sind diese Nadeln 25 in der Figur 2.6 zu erkennen. Sie haben an ihrem freien Ende jeweils einen Widerhaken und dringen damit in das Gewebe des Ohres 14 ein, wenn dieses, wie zum Beispiel die Figur 2.3 zeigt, gegen den Kragen 24 gedrückt wird.

Wird der Dorn weiter abwärts gedrückt, so wird, wie Figur 2.4 zeigt, der Kragen 24, der elastisch in der Entnahmeöffnung 19 sitzt, nach unten aus dieser herausgedrückt und dabei wird die von seinen Nadeln 25 erhaltene Probe 22 vom Ohr 16, welches oberhalb der Entnahmeöffnung 19 bleibt, endgültig abgerissen. Bei weiterer Abwärtsbewegung des Dornes 8 gemäß Figur 2.5 wird der Kragen 24 mit der Probe 22 vom Dorn 8 zur Seite bewegt und im Entnahmeraum 18 abgelegt. Figur 2.6 zeigt eine weit abgesenkte Stellung des Dornes 8. Dieser hat nun auch den Entnahmekopf 17 in seiner Führung 21 zur Seite bewegt. Dabei ist der Dornschaft 9 seitlich durch den Schlitz 23 ausgetreten. Die Probe kann nun zusammen mit dem Entnahmekopf 17 von der Zange abgenommen werden.

Vergleicht man die erste Ausführungsform der Figuren 1.1 bis 1.7 mit der zweiten Ausführungsform der Figuren 2.1 bis 2.6, so sieht man, dass der wesentliche Unterschied darin besteht, wie die Probe 22 vom Ohr 16 abgetrennt wird, nämlich in einem Fall im Wesentlichen durch Abschneiden und im anderen Fall im Wesentlichen durch Abreißen. Diese beiden Möglichkeiten können gegebenenfalls auch miteinander kombiniert werden.

Beiden Ausführungsformen gemeinsam ist das Abtrennen der Probe 22 vom Ohr 16 durch eine Kollision des Dornes 8 mit der Entnahmeöffnung 19 des Entnahmkopfes 17. Dazu kommt es, nachdem der Dorn 8 das Ohr 16 durchlaufen hat. Der Entnahmekopf 17 ist dazu auf der zum Loch 12 hin gelegenen Seite des Ohres 16 in der Nähe des Loches 12 angeordnet. Die Probe 22 würde an sich dem Dorn 8 im Wege liegen und würde von diesem durch das Loch 12 bis in die Kammer 13 mitgenommen werden. Gemäß der Erfindung wird jedoch der Probe 22 durch Interaktion zwischen dem Dorn 8 mit dem Entnahmekopf 17 eine Seitwärtskomponente der Bewegung vermittelt, so dass die Probe 22 im Endeffekt im Entnahmeraum 18 im Entnahmekopfes 17 landet und nur der Dorn 8 , in der Kammer 13.

Bei den bisher diskutierten Ausführungsformen erfolgt das Abtrennen der Probe 22 im Wesentlichen durch Interaktion des Dornes 8 mit der Entnahmeöffnung 19. Berücksichtigt man dabei die konzentrische Geometrie der Anordnung des Dornes zur Entnahmeöffnung 19, so könnte dabei eine Probe entstehen, die als Ring den Dorn umgibt. Das könnte die ungünstige Tendenz ergeben, dass der Dorn 8 die Probe bis in die Kammer 13 mitnimmt. Daher sind nicht gezeigte Einrichtungen vorteilhaft, die beim weiteren Vordringen des Dornes diesen Ring aufschneiden, damit er sicher vom Dorn getrennt wird.

Im übrigen kann die Probe durchaus auch zunächst nur teilweise abgetrennt werden und noch für einen Zeitraum, z.B. bis zum Ende der Querbewegung, mit dem Ohr über einen dünnen Gewebestrang verbunden bleiben. Der Gewebestrang kann z.B. verwendet werden, um eine ringförmig auf dem Dorn sitzende Probe von diesem abzuziehen. Der Gewebestrang kann schliesslich endgültig abgetrennt werden. Dabei kann die zunächst nur teilweise Abtrennung mit einer ersten Trenneinrichtung erfolgen und für die endgültige Abtrennung eine zweite Trenneinrichtung vorgesehen sein.

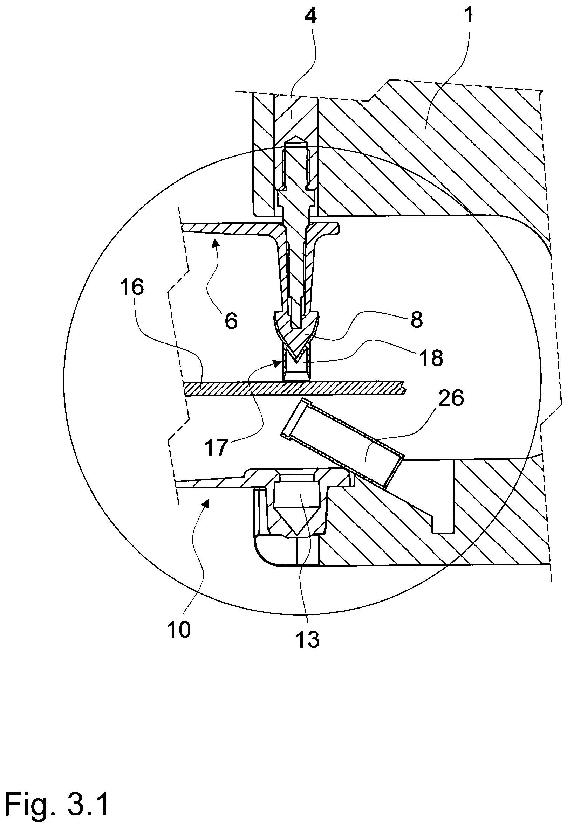

Die Figuren 3.1 bis 3.4 zeigen eine dritte Ausführungsform der Erfindung, die sich etwas weiter von den vorher ergehenden Ausführungsformen unterscheidet. Die meisten Teile bleiben jedoch unverändert und sind mit denselben Bezugszeichen versehen.

Wie Figur 3.1 zeigt, ist abweichend von den vorhergehenden Ausführungsformen der Entnahmekopf 17 unmittelbar in der Achse des Dornes 8 in dessen Bewegungsrichtung vor diesem angeordnet und dazu mit einer trichterförmigen Führung auf der konischen Außenfläche des Dornes 8 formschlüssig gehalten. Der Entnahmekopf 17 weist wiederum den Entnahmeraum 18 auf und die Entnahmeöffnung 19, die in Bewegungsrichtung des Dornes 8 offen ist und eine Ringschneide ausbildet.

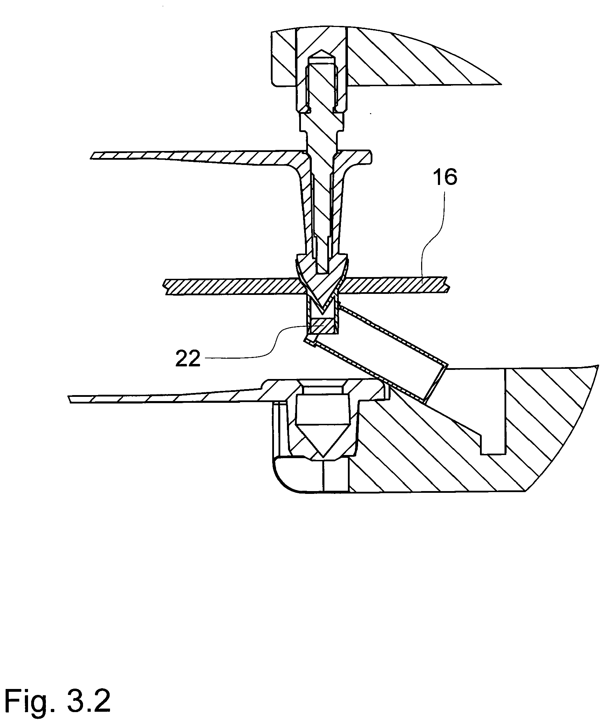

Beim Durchdringen des Ohres wird, wie Figur 3.2 zeigt, von der Ringschneide, die Probe 22 abgeschnitten und im Entnahmeraum 18 des Entnahmekopfes 17 gehalten.

Man sieht hier die konventionelle Anordnung, wie zum Beispiel in der eingangs zur Gattung zitierten Druckschrift gezeigt.

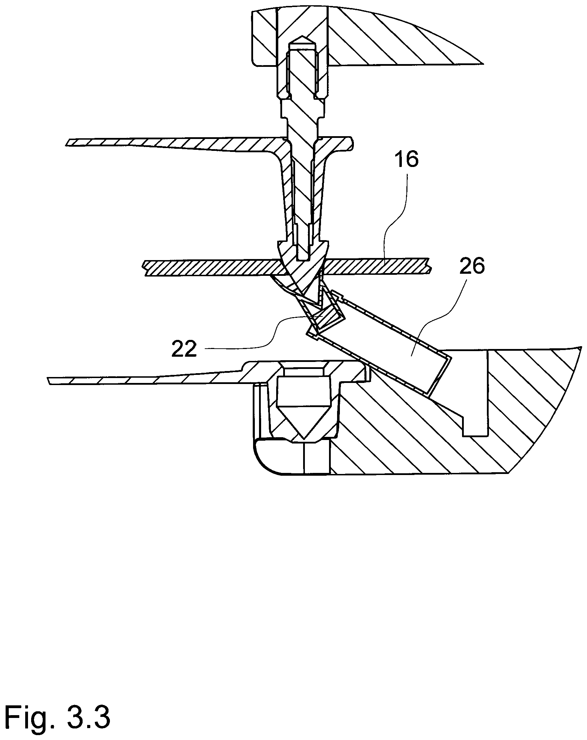

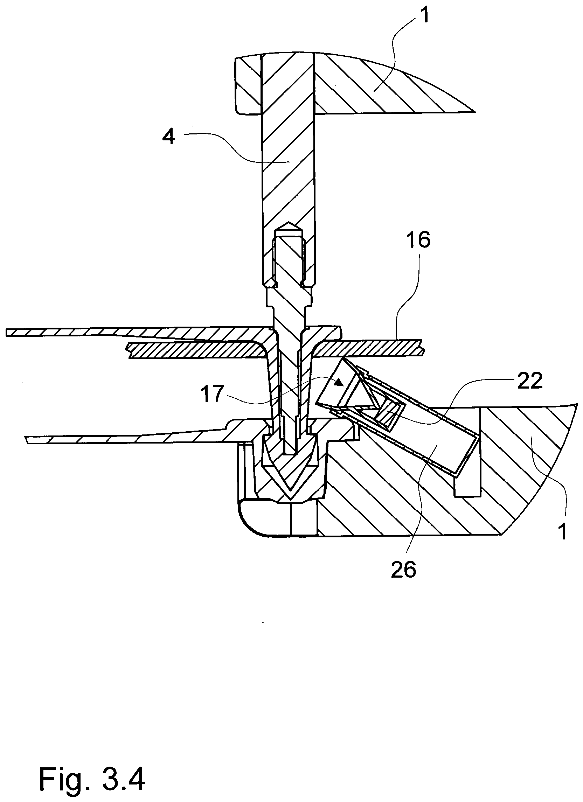

Anders als bei dieser, wird aber der Entnahmekopf 17 nicht bis in die Kammer 13 befördert, sondern, kommt wie Figur 3.2 zeigt, noch vor Erreichen des Loches 12 in die Öffnung eines Containers 26, der schräg stehend oberhalb des Loches 12 gehalten ist. Wie Figur 3.3 zeigt, wird bei weiterer Abwärtsbewegung des Dornes 8 der gegen die Wand des Containers 26 laufende Entnahmekopf 17 schräg abgekippt und vom weiter abwärts laufenden Dorn 8 in den Container hinein gedrückt. Bei weiterer Abwärtsbewegung wird schließlich auch der Container 26 zur Seite gedrückt bis er auf einer Schrägführung 27, die in Figur 3.3 dargestellt ist, an einen Anschlag gelangt, wie in Figur 3.4 dargestellt ist. Von da kann der Container entnommen werden, der bei entsprechender Ausbildung des Entnahmekopfes 17 von diesem luftdicht verschließbar ist. Er kann als Transportcontainer bis zum Labor dienen.

Auch hier wird also die Probe 22 nach ihrer Abtrennung vom Ohr seitlich gegenüber der Bewegungsbahn des Dornes 8 bewegt. Bei dieser Ausführungsform der Figur 3.1 bis 3.4, wird allerdings die Probe 22 zusammen mit dem Entnahmekopf 17 bewegt.

Bei den bisher diskutierten ersten drei Ausführungsformen der Erfindung, wird nur der Dorn 8 angetrieben. Daraus wird in Zusammenenarbeit mit dem Entnahmekopf 17 die Abtrennung generiert und die abgetrennte Probe 22 sodann seitlich bewegt.

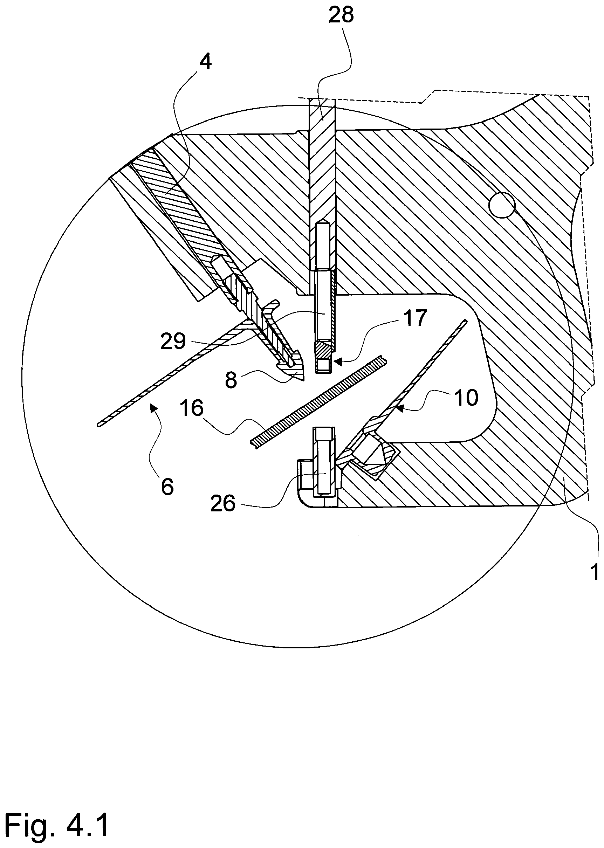

Die Figuren 4.1 bis 4.3 zeigen eine vierte Ausführungsform der Erfindung, mit etwas anderer Kinematik. Es werden wiederum soweit möglich die bisherigen Bezugszeichen verwendet.

Die Zange 1 führt wiederum den Bolzen 4 zum Antrieb des Dornes 8. An der Zange 1 ist unter einem Winkel zum Bolzen 4 ein zweiter Bolzen 28 gelagert, der mit einer nicht dargestellten kinematischen Verbindung an den Antrieb des Bolzens 4 gekoppelt ist. Der zweite Bolzen 28 hält ein Rohr 29, an dessen Ende der Entnahmekopf 17 befestigt ist, der entsprechend der Konstruktion der dritten Ausführungsform eine stirnseitige Rohrschneide aufweist.

Vergleicht man die Bewegungen des Entnahmekopfes 17, so sieht man, dass bei Abwärtsbewegung des Bolzens 28 der Entnahmekopf 17 zunächst mit seiner stirnseitigen Ringschneide, das Ohr 16 durchstanzt und dann in den Container 26 gesteckt wird, der im Fußteil der Zange 1 gehalten ist. Der Entnahmekopf 17 kann den Container abdichtend verschließen, wonach die im Entnahmekopf 17 gelagerte Probe 22 im Inneren des Containers 26 luftdicht verschlossen ist.

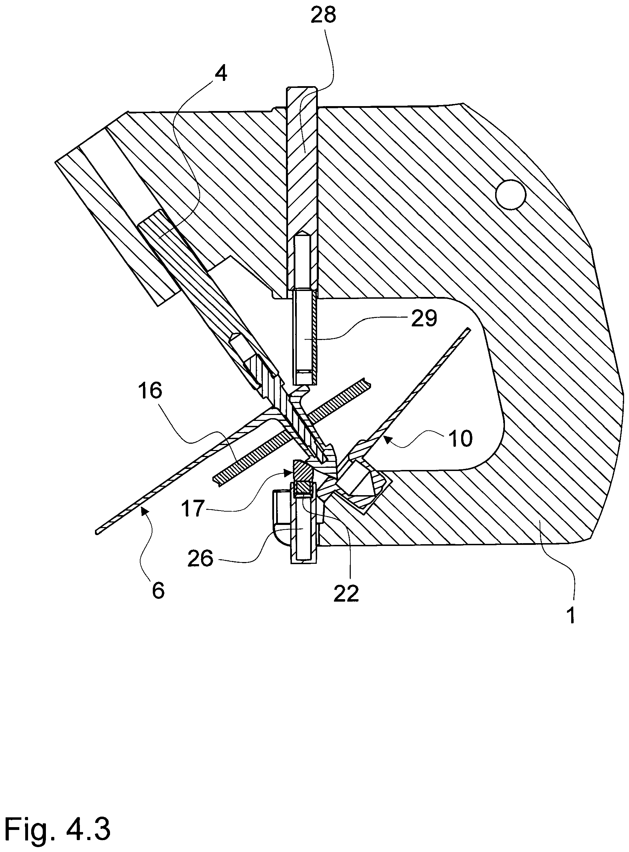

Anschließend kann der Bolzen 28 wieder nach oben gezogen werden, wie dies die Figur 4.3 zeigt.

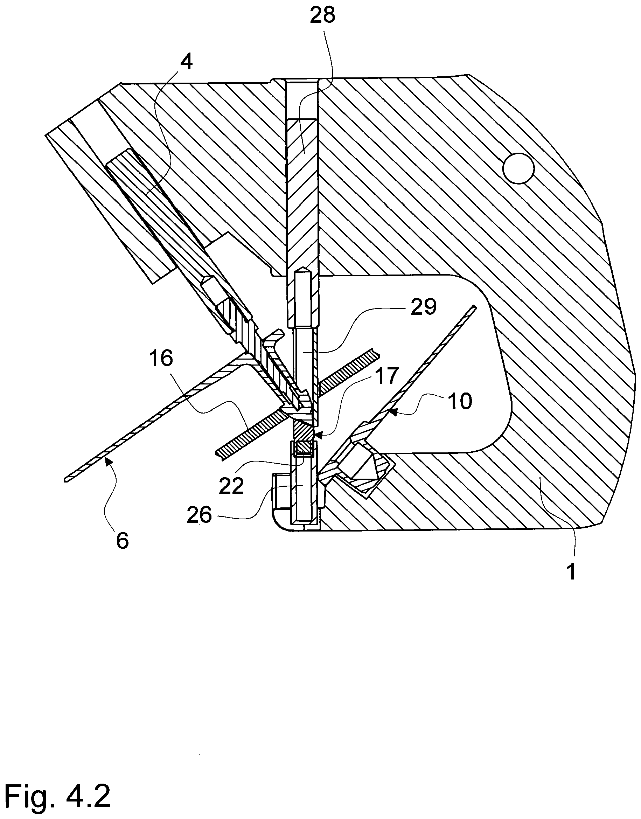

Der Dorn 8 wird bei dieser Ausführungsform der Zange getrennt vom Entnahmekopf 17 angetrieben, ist jedoch mit diesem über eine Kinematik bewegungsgekoppelt. In Figur 4.1 steht der Dorn 8 noch außer Eingriff. Er soll aber das Ohr 16 durchlaufen und zwar an derselben Stelle, an der zuvor von der Ringschneide des Entnahmekopfes 17 ein Loch geschnitten wurde. Daher muss der Dorn 8 in seiner Bewegung etwas hinter der Bewegung des Entnahmekopfes 17 hinterher laufen, so dass der Entnahmekopf 17 das Loch im Ohr 16 vollständig durchlaufen hat, bevor der Dorn 8 ankommt. Dieser Moment ist in Figur 4.2 dargestellt. Der Entnahmekopf 17 steht schon unterhalb des Ohres, sodass der Dorn 8 nun das Loch im Ohr 16 passieren kann. In der dargestellten Ausführungsform durchdringt er dabei das Rohr 29, wie diese Figur 4.2 zeigt. Figur 4.3 zeigt den Dorn kurz vor Erreichen seiner Endstellung im Lochteil 10 der Ohrmarke.

Wie man bei dieser vierten Ausführungsform sieht, erfährt auch hier die abgetrennte Probe 22 eine gegenüber der Bewegungsbahn des Dornes 8 seitlich gerichtete Bewegungskomponente.

## Patentansprüche

1. Verfahren zur Anbringung einer Ohrmarke (6, 10) an einem Ohr (16) und zum Abtrennen einer Gewebe des Ohres enthaltenden Probe (22) von diesem, **dadurch gekennzeichnet dass**,
ein an einem Dornbereich (6) der Ohrmarke befestigter Dorn (8) mittels einer Zange (1) auf einer ersten Bewegungsbahn durch das Ohr (16) in ein an einem Lochteil (10) der Ohrmarke ausgebildetes Loch (12) gebracht und dort verankert wird, währenddessen mit Hilfe eines Entnahmekopfes (17) die Probe (22) gewonnen und durch Zusammenarbeit mit dem Dorn (8) mit einer quer zur ersten Bewegungsbahn gerichtete Bewegungskomponente ausser Eingriff mit dem Loch (12) gebracht wird, bevor der Dorn (8) dort verankert wird.

2. Verfahren nach Anspruch 1, **dadurch gekennzeichnet, dass** der Dorn (8) beim Durchdringen des Ohres (16) dieses mit dem Bereich der Probe (22) gegen einen zur Aufnahme der Probe (22) ausgebildeten Behälter (17, 26) drückt und dabei die Probe (22) abtrennt.

3. Verfahren nach Anspruch 2, **dadurch gekennzeichnet, dass** das Abtrennen durch Abschneiden erfolgt.

4. Verfahren nach Anspruch 2 oder 3, **dadurch gekennzeichnet, dass** das Abtrennen durch Abreissen erfolgt.

5. Verfahren nach Anspruch 1, **dadurch gekennzeichnet, dass** der Dorn (8) auf seinem Weg zum Loch (12) den Entnahmekopf (17) vor sich her schiebt, dann unter Entnahme der Probe (22) durch das Ohr (16) hindurch schiebt und dann gegen eine Einrichtung (26, 27) schiebt, die die Probe seitlich ablenkt.

6. Verfahren nach Anspruch 1, **dadurch gekennzeichnet, dass** der Entnahmekopf (17) getrennt vom Dorn (8) auf einer Bahn bewegt wird, die in einem Winkel zur ersten Bewegungsbahn steht und diese dann schneidet, wenn der Entnahmekopf (17) das Ohr (16) durchdringt.

7. Vorrichtung zur Durchführung des Verfahrens nach einem der Ansprüche 1 bis 6, mit einem an einem Dornbereich (6) der Ohrmarke befestigten Dorn (8), der mittels einer Zange (1) auf einer durch das Ohr (16) verlaufenden ersten Bewegungsbahn in ein an einem Lochteil (10) der Ohrmarke ausgebildetes Loch (12) bringbar und dort verankerbar ist, und mit einem zur Entnahme der Probe (22) ausgebildeten Entnahmekopf (17), **dadurch gekennzeichnet, dass** der Entnahmekopf (17) mit dem Dorn (8) derart zusammenarbeitend angeordnet und ausgebildet ist, dass bei der Bewegung des Dornes (8) bis zur Verankerungsposition im Loch (12) die Probe (22) in einer zweiten Bewegungsbahn läuft, die wenigstens in einem vor dem Loch angeordneten Querbereich eine quer zur ersten Bewegungsbahn gerichtete Bewegungskomponente aufweist.

8. Vorrichtung nach Anspruch 7, **gekennzeichnet durch** eine Trenneinrichtung, die zum Abtrennen zusammenwirkende Trennglieder (8, 17, 26) aufweist, von denen eines (17, 26) im Bereich des Lochteiles (10) in der Bewegungsbahn des Dornes (8) vor dem Loch (12) steht und ein anderes (8) in der Bewegungsbahn des Dornes (8) bewegt ist.

9. Vorrichtung nach Anspruch 8, **dadurch gekennzeichnet, dass** die Trennglieder (8, 17, 26) derart zusammenwirkend ausgebildet sind, dass bei dem Zusammenwirken die quer gerichtete Bewegungskomponente der Probe (22) entsteht.

10. Vorrichtung nach Anspruch 8 oder 9, **dadurch gekennzeichnet, dass** der Dorn (8) das bewegte Trennglied ausbildet.

11. Vorrichtung nach einem der Ansprüche 8 bis 10, **dadurch gekennzeichnet, dass** das stehende Trennglied einen zur Aufnahme der Probe ausgebildeten Behälter (17, 26) bildet.

12. Vorrichtung nach Anspruch 11, **dadurch gekennzeichnet, dass** der Behälter den Entnahmekopf (17) ausbildet.

13. Vorrichtung nach einem der Ansprüche 8 bis 11, **dadurch gekennzeichnet, dass** der Dorn (8) mit einer schräg zu seiner Bewegungsbahn verlaufenden Fläche ausgebildet ist.

14. Vorrichtung nach Anspruch 13, **dadurch gekennzeichnet, dass** die Fläche eine Kegelfläche ist.

15. Vorrichtung nach einem der Ansprüche 12 bis 14, **dadurch gekennzeichnet, dass** der Entnahmekopf (17) eine vom Dorn (8) kontaktierbare Schneide (19) aufweist.

16. Vorrichtung nach einem der Ansprüche 12 bis 15, **dadurch gekennzeichnet, dass** der Entnahmekopf (17), eine vom Dorn kontaktierbare Nadelanordnung (24, 25) aufweist.

17. Vorrichtung nach einem der Ansprüche 12 bis 16, **dadurch gekennzeichnet, dass** der Entnahmekopf (17) einen parallel zur Bewegungsbahn des Dornes (8) ausgerichteten Schlitz (23) aufweist.

18. Vorrichtung nach einem der Ansprüche 7 bis 9, **dadurch gekennzeichnet, dass** der Entnahmekopf (17) am Dorn (8), in Bewegungsrichtung vor diesem, angeordnet ist und dass im Bereich des Lochteiles (10), eine Einrichtung (26, 27) zum seitlichen Ablenken des Entnahmekopfes (17) angeordnet ist.

19. Vorrichtung nach Anspruch 7, **dadurch gekennzeichnet, dass** der Entnahmekopf (17) in der zweiten Bewegungsbahn geführt ist, die schräg zur ersten Bewegungsbahn derart angeordnet ist, dass sie die erste Bewegungsbahn am Punkt der Durchdringung des Ohres (16) schneidet, wobei der Entnahmekopf (17) mit dem Dorn (8) derart zusammen arbeitet, dass er den Punkt der Durchdringung zeitlich vor dem Dorn (8) erreicht.

20. Entnahmekopf einer Vorrichtung nach einem der Ansprüche 7 bis 19.

## Claims

1. A method for attaching an ear tag (6, 10) to an ear (16) and for severing a sample (22) containing tissue of the ear therefrom, **characterized in that** a mandrel (8), which is fixed to a mandrel region (6) of the ear tag, is brought by means of applicator pliers (1) on a first movement path through the ear (16) into a hole (12) formed in a hole part (10) of the ear tag and anchored there, while the sample (22) is obtained with the aid of a removal head (17) and is brought out of engagement with the hole (12) by interaction with the mandrel (8) with a movement component oriented transversely with respect to the first movement path before the mandrel (8) is anchored in said hole.

2. The method according to claim 1, **characterized in that**, on penetrating the ear (16), the mandrel (8) presses it with the region of the sample (22) against a container (17, 26) designed to accommodate the sample (22) and in doing so severs the sample (22).

3. The method according to claim 2, **characterized in that** the severing action occurs by cutting.

4. The method according to claim 2 or 3, **characterized in that** the severing action occurs by tearing.

5. The method according to claim 1, **characterized in that** the mandrel (8) pushes the removal head (17) in front of it on its way to the hole (12), then pushes it through the ear (16) while removing the sample (22), and then pushes it against a device (26, 27) which deflects the sample to the side.

6. The method according to claim 1, **characterized in that** the removal head (17) is moved separately from the mandrel (8) on a path which is at an angle to the first movement path and then cuts this when the removal head (17) penetrates the ear (16).

7. A device for carrying out the method according to any one of claims 1 to 6, having a mandrel (8) fixed to a mandrel region (6) of the ear tag, which mandrel can be brought by means of applicator pliers (1) on a first movement path running through the ear (16) into a hole (12) formed in a hole part (10) of the ear tag and anchored there, and having a removal head (17) designed to remove the sample (22), **characterized in that** the removal head (17) is arranged and designed to interact with the mandrel (8) in such a way that, on moving the mandrel (8) to the anchoring position in the hole (12), the sample (22) runs in a second movement path which, at least in a transverse region arranged before the hole, has a movement component oriented transversely with respect to the first movement.

8. The device according to claim 7, **characterized by** a severing device which has severing members (8, 17, 26) which interact for the purpose of severing, of which one (17, 26), in the region of the hole part (10), is located before the hole (12) in the movement path of the mandrel (8), and another (8) is moved in the movement path of the mandrel (8).

9. The device according to claim 8, **characterized in that** the severing members (8, 17, 26) are designed to interact in such a way that the transversely oriented movement component of the sample (22) is produced by the interaction.

10. The device according to claim 8 or 9, **characterized in that** the mandrel (8) forms the moving severing member.

11. The device according to one of claims 8 to 10, **characterized in that** the fixed severing member forms a container (17, 26) designed to accommodate the sample.

12. The device according to claim 11, **characterized in that** the container forms the removal head (17).

13. The device according to any one of claims 8 to 11, **characterized in that** the mandrel (8) is designed with a surface which runs at an angle to its movement path.

14. The device according to claim 13, **characterized in that** the surface is a conical surface.

15. The device according to any one of claims 12 to 14, **characterized in that** the removal head (17) has a cutter (19) which can be contacted by the mandrel (8).

16. The device according to any one of claims 12 to 15, **characterized in that** the removal head (17) has a needle arrangement (24, 25) which can be contacted by the mandrel.

17. The device according to any one of claims 12 to 16, **characterized in that** the removal head (17) has a slot (23) oriented parallel to the movement path of the mandrel (8).

18. The device according to any one of claims 7 to 9, **characterized in that** the removal head (17) is arranged on the mandrel (8) before said mandrel in the direction of movement and that a device (26,27) for deflecting the removal head (17) to the side is arranged in the region of the hole part (10).

19. The device according to claim 7, **characterized in that** the removal head (17) is guided in the second movement path, which is arranged at an angle to the first movement path in such a way that it cuts the first movement path at the point of penetration of the ear (16), wherein the removal head (17) works together with the mandrel (8) in such a way that it reaches the point of penetration at a time before the mandrel (8).

20. A removal head of a device according to any one of claims 7 to 19.

## Revendications

1. Procédé pour poser une marque auriculaire (6, 10) sur une oreille (16) et pour prélever de celle-ci un échantillon (22) contenant du tissu auriculaire, **caractérisé en ce qu'**un mandrin (8) fixé sur une zone de mandrin (6) de la marque auriculaire, est introduit, au moyen d'une pince (1) sur une première voie de déplacement à travers l'oreille (16), dans un trou (12) formé dans une partie de trou (10) de la marque auriculaire et y est ancré, tandis que, à l'aide d'une tête de prélèvement (17), l'échantillon (22) est obtenu et désengagé par coopération avec le mandrin (8) avec un composant de déplacement orienté transversalement à la première voie de déplacement d'avec le trou (12) avant que le mandrin (8) y soit ancré.

2. Procédé selon la revendication 1, **caractérisé en ce que** lorsque le mandrin (8) pénètre dans l'oreille (16), il appuie sur celle-ci avec la zone de l'échantillon (22) contre un récipient (17, 26) conçu pour recevoir l'échantillon (22) et prélève ainsi l'échantillon (22).

3. Procédé selon la revendication 2, **caractérisé en ce que** le prélèvement s'effectue par sectionnement.

4. Procédé selon la revendication 2 ou 3, **caractérisé en ce que** le prélèvement s'effectue par arrachement.

5. Procédé selon la revendication 1, **caractérisé en ce que** le mandrin (8) pousse la tête de prélèvement (17) devant lui sur sa trajectoire vers le trou (12) puis la pousse à travers l'oreille (16) tout en prélevant l'échantillon (22) puis la pousse contre un dispositif (26, 27) qui dévie latéralement l'échantillon.

6. Procédé selon la revendication 1, **caractérisé en ce que** la tête de prélèvement (17) est déplacée séparément du mandrin (8) sur une voie qui se situe à un certain angle par rapport à la première voie de déplacement puis la coupe lorsque la tête de prélèvement (17) pénètre dans l'oreille (16).

7. Dispositif pour réaliser le procédé selon l'une des revendications 1 à 6, ayant un mandrin (8) fixé sur une zone de mandrin (6) de la marque auriculaire, lequel mandrin peut être introduit au moyen d'une pince (1) sur une première voie de déplacement s'étendant à travers l'oreille (16) dans un trou (12) formé dans une partie de trou (10) de la marque auriculaire et peut y être ancré et ayant une tête de prélèvement (17) conçue pour prélever l'échantillon (22), **caractérisé en ce que** la tête de prélèvement (17) est agencée et conçue pour interagir avec le mandrin (8) de manière à ce qu'en déplaçant le mandrin (8) en position d'ancrage dans le trou (12), l'échantillon (22) se déplace sur une seconde voie de déplacement qui, au moins dans une zone transversale agencée avant le trou, présente un composant de déplacement orienté transversalement par rapport à la première voie de déplacement.

8. Dispositif selon la revendication 7, **caractérisé par** un dispositif de prélèvement qui présente plusieurs éléments coupants (8, 17, 26) qui interagissent à des fins de coupe, dont l'un (17, 26), dans la zone de la partie de trou (10), est situé avant le trou (12) sur la voie de déplacement du mandrin (8) et l'autre (8) est déplacé sur la voie de déplacement du mandrin (8).

9. Dispositif selon la revendication 8, **caractérisé en ce que** les éléments coupants (8, 17, 26) sont conçus pour interagir de manière à ce que le composant de déplacement orienté transversalement de l'échantillon (22) soit produit par interaction.

10. Dispositif selon la revendication 8 ou 9, **caractérisé en ce que** le mandrin (8) forme l'élément coupant mobile.

11. Dispositif selon l'une des revendications 8 à 10, **caractérisé en ce que** l'élément coupant fixe forme un récipient (17, 26) conçu pour recevoir l'échantillon.

12. Dispositif selon la revendication 11, **caractérisé en ce que** le récipient forme la tête de prélèvement (17).

13. Dispositif selon l'une des revendications 8 à 11, **caractérisé en ce que** le mandrin (8) est conçu avec une surface s'étendant à l'oblique par rapport à sa voie de déplacement.

14. Dispositif selon la revendication 13, **caractérisé en ce que** la surface est une surface conique.

15. Dispositif selon l'une des revendications 12 à 14, **caractérisé en ce que** la tête de prélèvement (17) présente un élément coupant (19) qui peut venir en contact avec le mandrin (8).

16. Dispositif selon l'une des revendications 12 à 15, **caractérisé en ce que** la tête de prélèvement (17) présente un agencement d'aiguilles (24, 25) qui peut venir en contact avec le mandrin.

17. Dispositif selon l'une des revendications 12 à 16, **caractérisé en ce que** la tête de prélèvement (17) présente une fente (23) orientée parallèlement à la voie de déplacement du mandrin (8).

18. Dispositif selon l'une des revendications 7 à 9, **caractérisé en ce que** la tête de prélèvement (17) est agencée sur le mandrin (8) avant celui-ci dans le sens de déplacement et **en ce qu'**un dispositif (26, 27) pour dévier latéralement la tête de prélèvement (17) est agencé dans la zone de la partie de trou (10).

19. Dispositif selon la revendication 7, **caractérisé en ce que** la tête de prélèvement (17) est guidée sur la seconde voie de déplacement, qui est agencée à l'oblique par rapport à la première voie de déplacement de manière à couper la première voie de déplacement au point de pénétration dans l'oreille (16), la tête de prélèvement (17) agissant conjointement avec le mandrin (8) de manière à atteindre le point de pénétration à un moment avant le mandrin (8).

20. Tête de prélèvement d'un dispositif selon l'une des revendications 7 à 19.
